# EUROPÄISCHE PATENTSCHRIFT

(11) **EP 0 423 137 B1**
(45) Veröffentlichungstag und Bekanntmachung des Hinweises auf die Patenterteilung: **09.02.1994**
(21) Anmeldenummer: 89905101.5
(22) Anmeldetag: 27.04.1989
(51) Int. Cl.: A01N 59/00, C01B 11/02

(54) **WÄSSRIGE LÖSUNG ZUR OXIDATION, ENTKEIMUNG UND DESODORIERUNG SOWIE VERFAHREN ZUR HERSTELLUNG DIESER LÖSUNG**
AQUEOUS OXIDIZING, DISINFECTING AND DEODORISING SOLUTION, AND PROCESS FOR PRODUCING SAID SOLUTION
SOLUTION AQUEUSE D'OXIDATION, DE DESINFECTION ET DE DESODORISATION ET SON PROCEDE DE FABRICATION

(30) Priorität: 06.05.1988 DE 3815456
(43) Veröffentlichungstag der Anmeldung: 24.04.1991
(73) Patentinhaber: CEALIN CHEMISCHE FABRIK GMBH, D-31157 Sarstedt (DE)
(72) Erfinder: LEHMANN, Wilfried, D-3203 Sarstedt (DE)
(74) Vertreter: König, Norbert, Dipl.-Phys. Dr.
(86) Internationale Anmeldenummer: EP8900462
(87) Internationale Veröffentlichungsnummer: WO8910695

(56) Entgegenhaltungen:
- CA-A- 965 699
- DE-B- 1 259 022
- GB-A- 2 176 812
- US-A- 4 737 307
- Seifen-Öle-Fette-Wachse, volume 94, No. 11, 22 May 1968, (Augsburg, DE), H. Hadert: "Aktive Chlorverbindungen", pages 329-332, see page 330, lines 32-40

## Beschreibung

Die Erfindung betrifft eine wäßrige Lösung zur Oxidation, Entkeimung und Desodorierung sowie ein Verfahren zur Herstellung einer solchen wäßrigen Lösung.

Bisher ist es bekannt, für die Oxidation, Entkeimung und Desodorierung Chlorlösungen einzusetzen. Nachteilig ist, daß das Chlor dieser Lösungen mit den Bestandteilen, beispielsweise des zu behandelnden Abwassers, Zwischenverbindungen eingeht, wobei diese Zwischenverbindungen Chlorstickstoffverbindungen, Chlorsubstitutionsverbindungen, Chlorphenole, halogenierte Chlorkohlenwasserstoffe, Trihalomethane, Chloramine und andere sind. Durch das Eingehen von Zwischenverbindungen steht beispielsweise für die Entkeimung nicht mehr genügend freies Chlor zur Verfügung, so daß die Entkeimungswirkung gering ist und zur Erzielung einer ausreichenden Entkeimung relativ große Mengen an Chlorlösung eingesetzt werden müssen.

Durch die GB-A-2 176 812 ist ein Verfahren zum Delignifizieren und Bleichen von Zellulosematerial bekannt, bei dem das Zellulosematerial in Gegenwart einer der Säuren Kohlensäure, Essigsäure, Salpetersäure, Salzsäure, Schwefelsäure oder Phosphorsäure mit Chlordioxid in Kombination mit Hypochlorit behandelt wird, wobei eine Chlordioxidlösung und eine Hypochloritlösung jeweils als separate Lösungen gleichzeitig zugegeben werden.

Aus der Zeitschrift "Seifen-öle-Fette-Wachse"-94.Jg. - Nr. 11/1968, S. 329 bis 332 ist eine Bleichlösung mit der Bezeichnung "Eau de Javelle" bekannt, die dadurch hergestellt wird, daß Chlor in eine Lösung von Natrium- oder Kaliumkarbonat eingeleitet wird. Diese Lösung besteht vorwiegend aus Unterchlorigsäure und etwas Natrium- oder Kaliumhypochlorit.

Der Erfindung liegt die Aufgabe zugrunde, eine verbesserte, die obigen Nachteile nicht mehr aufweisende wäßrige Lösung zur Oxidation, Entkeimung und Desodorierung sowie ein Verfahren zur Herstellung dieser Lösung anzugeben.

Diese Aufgabe wird durch die im Anspruch 1 angegebene wäßrige Lösung gelöst. Ein Verfahren zur Herstellung dieser Lösung ist im Anspruch 5 angegeben. Zweckmäßige Weiterbildungen sind in den weiteren Ansprüchen angegeben.

Die erfindungsgemäße wäßrige Lösung enthält außer Natriumhypochlorit und/oder Magnesiumhypochlorit und/oder Kalziumhypochlorit die Chlorsauerstoffsäuren HClO₂, HClO₃ und/oder HClO₄ und/oder die Salze dieser Säuren.

Die erfindungsgemäße wäßrige Lösung zur Oxidation, Entkeimung und Desodorierung weist einen relativ hohen Aktivchlorgehalt auf. Die Lösung vereinigt die Vorteile von Chlor und den eingesetzten Chlorsauerstoffsäuren und/oder deren Salzen dieser Säuren.

Beim Einsatz der erfindungsgemäß hergestellten wäßrigen Lösung entstehen die oben erwähnten Zwischenverbindungen nicht mehr. Es steht nur freies wirksames Chlor zur Verfügung, so daß wesentlich kürzere Entkeimungszeiten bei wesentlich geringeren Einsatzmengen erreicht werden.

Die mit dem erfindungsgemäßen Verfahren hergestellte wäßrige Lösung weist folgende Vorteile auf:
a) Abtötung von Mikroorganismen: Durch Oxidation werden Keime, Bakterien, Pilze und Algen abgetötet; Viren werden inaktiviert.
b) Geruchsheseitigung: Durch Oxidation geruchsbildender Verbindungen, wie z. B. Schwefelwasserstoff.
c) Entgiftung: Durch Oxidation von z. B. Nitrit in Nitrat.
d) Entschwefelung: Durch Oxidation von Schwefelverhindungen, z. B. Sulfide, Sulfite in Sulfate.
e) Entfernung von Eisen, Mangan und anderen Metallen: Durch Oxidation werden Metallkationen in die maximale Wertigkeit überführt, wobei diese je nach pH-Wert als unlösliche Oxide oder Hydroxide ausfallen bzw. filtrierfähig sind.
f) Senkung des Kalitumpermanganatverbrauchs: Durch Oxidation von wasserbelastenden Stoffen.
g) Redoxpotentialerhöhung: Durch Einbringung eines hohen Oxidationspotentials.
h) Biozide Wirkung: Resultiert aus der Oxidation der Zellkernbestandteile (Zytoplasma).
i) Algizide Wirkung: Ergibt sich aus der Oxidation des Chlorophylls.

Besonders vorteilhaft ist, daß die erfindungsgemäß hergestellte wäßrige Lösung im gesamten pH-Bereich ohne Einschränkung einsetzbar ist.

Die Erfindung soll anhand eines Beispiels näher erläutert werden.

In eine Natriumhypochloritlösung mit 15 bis 20 Gew.-% Chlor werden bezogen auf diese Lösung 10 Gew.-% einer 20-gewichtsprozentigen wäßrigen Lösung von HClO₂ und HClO₄ unter gleichmäßigem Rühren hinzugegeben. Das Rühren erfolgt mit ca. 500 Umdrehungen pro Minute bei Raumtemperatur (20°C bis 25°C) über eine Reaktionszeit von 15 Minuten. Nach dieser Rührzeit von 15 Minuten ist der Vermischungsvorgang abgeschlossen. Die erhaltene Lösung weist einen Aktivchlorgehalt von 1 bis 60 Gew.-% auf.

## Patentansprüche

1. Wäßrige Lösung zur Oxidation, Entkeimung und Desodorierung mir einem Gehalt an Natriumhypochlorit und/oder Magnesiumhypochlorit und/oder Kalziumhypochlorit, **dadurch gekennzeichnet**, daß sie zusätzlich die Chlorsauerstoffsäuren HClO₂, HClO₃ und/oder HClO₄ und/oder die Salze dieser Chlorsauerstoffsäuren enthält.

2. Wäßrige Lösung nach Anspruch 1, **dadurch gekennzeichnet**, daß der Anteil an Chlorsauerstoffsäuren und/oder deren Salze 0,1 bis 50 Gew.-% beträgt.

3. Wäßrige Lösung nach Anspruch 1 oder 2, **dadurch gekennzeichnet**, daß sie einen Aktivchlorgehalt von 1 bis 60 Gew.% aufweist.

4. Lösung nach einem der vorhergehenden Ansprüche, **dadurch gekennzeichnet**, daß die Natriumhypochlorit-, Magnesiumhypochlorit- oder Kalziumhypochlorit-Lösung 1 bis 50 gewichtsprozentig ist.

5. Verfahren zur Herstellung einer wäßrigen Lösung zur Oxidation, Entkeimung und Desodorierung nach einem der Ansprüche 1 bis 4, **dadurch gekennzeichnet**, daß in die wäßrige Natriumhypochlorit-Lösung und/oder Magnesiumhypochlorit- und/oder Kalziumhypochlorit-Lösung 0,1 bis 50 Gew.-% der Chlorsauerstoffsäuren und/oder deren Salze unter Rühren eingegeben werden.

6. Verfahren nach Anspruch 5, **dadurch gekennzeichnet**, daß die unter Rühren durchgeführte Zugabe in einem abgeschlossenen Behälter erfolgt.

## Claims

1. An aqueous oxidizing, disinfecting and deodorising solution having a content of sodium hypochlorite and/or magnesium hypochlorite and/or chlorinated lime, **characterised in that** in addition it contains the chlorous or chloric acids HClO₂, HClO₃ and/or HClO₄ and/or the salts of these chlorous or chloric acids.

2. An aqueous solution according to claim 1,
**characterised in that** the content of chlorous or chloric acids and/or their salts is 0.1 to 50 % by weight.

3. An aqueous solution according to Claim 1 or 2,
**characterised in that** it comprises an active carbon content of 1 to 60 % by weight.

4. A solution according to one of the preceding Claims,
**characterised in that** the sodium hypochlorite, magnesium hypochlorite or chlorinated lime solution is 1 to 50 percent by weight.

5. A process for producing an aqueous oxidizing, disinfecting and deodorising solution according to one of claims 1 to 4,
**characterised in that** 0.1 to 50 % by weight of the chlorous or chloric acids and/or of their salts are added to the aqueous sodium hypochlorite solution and/or magnesium hypochlorite and/or chlorinated lime solution accompanied by agitation.

6. A process according to Claim 5,
**characterised in that** the addition performed accompanied by agitation occurs in a sealed container.

## Revendications

1. Solution aqueuse pour l'oxydation, la désinfection et la désodorisation avec un contenu d'hypochlorite de sodium et/ou d'hypochlorite de magnésium et/ou hypochlorite de calcium, caractérisée en ce qu'elle contient additionnellement les acides de chlore et oxygène HCl O₂, HCl O₃ et/ou HCl O₄ et/ou les sels de ces acides de chlore et d'oxygène.

2. Solution aqueuse selon la revendication 1,
caractérisée en ce que la portion d'acides de chlore et d'oxygène et/ou de leurs sels est de O,1 à 50% en poids.

3. Solution aqueuse selon la revendication 1 ou 2,
caractérisée en ce qu elle présente un contenu de chlore actif de 1 à 60% en poids.

4. Solution selon l'une des revendications précédentes,
caractérisée en ce que la solution d'hypochlorite de sodium, d'hypochlorite de magnésium ou d'hypochlorite de calcium est de 1 à 50 pour cent en poids.

5. Procédé pour la fabrication d'une solution aqueuse pour l'oxydation, la désinfection et la désodorisation selon l'une des revendications 1 à 4,
caractérisé en ce qu'on introduit sous agitation dans la solution d'hypochlorite de sodium et/ou la solution d'hypochlorite de magnésium et/ou d'hypochlorite de calcium de 0,1 à 50% en poids des acides de chlore et d'oxygène et/ou de leurs sels.

6. Procédé selon la revendication 5,
caractérisé en ce que l'addition effectuée sous agitation est effectuée dans un récipient scellé.
